# EUROPEAN PATENT SPECIFICATION

(11) **EP 3 849 441 B1**
(45) Date of publication and mention of the grant of the patent: **06.12.2023**
(21) Application number: 19859986.2
(22) Date of filing: 09.09.2019
(51) Int. Cl.: A61B 17/295, A61B 18/14, A61B 34/30

(54) **ARTICULATING BLADE DEPLOYMENT**
ENTFALTUNG EINER GELENKIGEN KLINGE
DÉPLOIEMENT DE LAME D'ARTICULATION

(30) Priority: 14.09.2018 US 201862731367 P
(43) Date of publication of application: 21.07.2021
(73) Proprietor: Covidien LP, Mansfield, MA 02048 (US)
(72) Inventor: HAMMERLAND, John, Arvada, Colorado 80007 (US); DROCHNER, Thomas, Longmont, Colorado 80504 (US)
(74) Representative: Maschio, Antonio
(86) International application number: PCT/US2019/050126
(87) International publication number: WO 2020/055705

(56) References cited:
- EP-A1- 2 732 779
- WO-A1-2018/067451
- US-A1- 2008 015 566

## Description

### BACKGROUND

Robotic surgical systems have been used in minimally invasive medical procedures. Some robotic surgical systems include a console supporting a robot arm, and at least one end effector such as forceps or a grasping tool that is mounted to the robot arm via a wrist assembly. During a medical procedure, the end effector and the wrist assembly are inserted into a small incision (via a cannula) or a natural orifice of a patient to position the end effector at a work site within the body of the patient.

In robotic surgical systems, cables extend from the robot console, through the robot arm, and connect to the wrist assembly and/or end effector. In some instances, the cables are actuated by means of motors that are controlled by a processing system including a user interface for a surgeon or clinician to be able to control the robotic surgical system including the robot arm, the wrist assembly and/or the end effector.

Existing wristed robotic instruments also have what is known as an elbowed design in which the end effector articulation point and the jaw pivot point are located at longitudinally spaced apart positions. Consequently, given the variety of positions in which these end effectors may be disposed to access surgical sites, one challenge associated with wristed robotic instruments is advancing blades through the end effectors to effectuate mechanical cutting when the end effector is disposed in one or more of the variety of positions.

EP2732779A1 relates to a bipolar coagulation instrument with first and second jaw members and a movable blade.

WO2018/067451 relates to a surgical instrument having an end effector component comprising a pair of jaw members and a translatable cutting element.

US2018/0036025A1 discloses an end effector assembly configured for use with a surgical instrument or surgical system includes first and second jaw members each including an outer jaw housing, a tissue-treating plate, and a longitudinally-extending channel therethrough. At least one of the first or second jaw members is pivotable relative to the other between a spaced-apart position and an approximated position. A cutting mechanism is configured to cut tissue grasped between the jaw members. The cutting mechanism may include an activation member coupled to a knife and configured to advance the knife transversely between the jaw members, a heat-activated elongated sheet configured to un-roll to advance a cutting edge thereof transversely between the jaw members, or a clutch and spring mechanism configured to deploy a knife longitudinally between the jaw members.

US2008/0015566A1 discloses an electrosurgical apparatus for sealing and cutting tissue includes forceps and a power supply. The forceps include jaws that are pivotal about a pin and at least one blade disposed between the jaws. A shaft connects a handle to the jaws for moving the jaws while also conducting electric current from the power supply through the jaws. A wire, routed through the shaft and through a hole in the pin, connects a lever to the blade for moving the blade. In a bipolar configuration, the wire also conducts the electric current through the blade.

US2016/0317172A1 discloses a tissue excision device comprising: a jaw disposed at a distal end of an insertion portion and used to sandwich tissue; a cutter movable along a track formed along the shape of the jaw; a drive unit which generates force to drive the cutter; and a force transmission member which connects the drive unit and the cutter and accommodated in an accommodation portion provided along the track, and which transmits the force generated by the drive unit to the cutter. The drive unit is configured to generate greater force as reaction force from the accommodation portion becomes greater.

### SUMMARY

Accordingly, the present disclosure details mechanical cutting solutions provided in a wristed design where articulation and jaw pivot exist at the same point. The invention relates to an end effector for a wristed surgical instrument as defined in claim 1. Further embodiments of the invention are specified in the dependent claims.

In accordance with one aspect, the present disclosure is directed to an end effector for a wristed surgical instrument. The end effector includes a first jaw member, a second jaw member coupled to the first jaw member by a pivot pin, an actuation string, and a blade supported between the first and second jaw members. The first and second jaw members are positioned to pivot and articulate about a pivot axis defined by the pivot pin. The blade includes a cam plate and is secured to the actuation string and movable relative to the first and second jaw members to sever tissue clamped between the first and second jaw members in response to actuation of the actuation string. The actuation string is secured to the cam plate. The second jaw member includes a cam plate and the pivot pin is positioned through the cam plates of the blade and the second jaw member.

In some embodiments, the actuation string may be routed through the cam plate of the blade. The cam plate of the second jaw member may include a ramp. The cam plate of the blade may be positioned to move along the ramp. The cam plate of the blade may define a ramp slot that receives the ramp of the second jaw member. The blade may be positioned to move vertically and axially relative to the second jaw member as the cam plate of the blade cams along the ramp of the second jaw member. The blade may include a cutting arm that extends distally from the cam plate of the blade. The blade may be configured to pivot relative to the second jaw member in response to actuation of the actuation string. The cutting arm may include a distal portion and a proximal portion. The distal portion of the cutting arm may be configured to move farther than the proximal portion of the cutting arm as the blade pivots relative to the second jaw member.

According to another aspect, the present disclosure is directed to a wristed surgical instrument including an end effector as defined above. The end effector defines a longitudinal axis and a pivot axis transverse to the longitudinal axis. The first jaw member and a second jaw member are movable about the pivot axis between an unclamped position and a clamped position. The actuation string is axially movable to actuate the blade relative to the first and second jaw members when the first and second jaw members are in the clamped position.

In certain embodiments, the end effector may be coupled to an electrosurgical energy source.

In some embodiments, the blade may be configured to move from the second jaw member toward the first jaw member to cut tissue. The blade may be vertically and axially movable relative to the first and second jaw members. The blade may cam along a ramp of the second jaw member in response to axial translation of the actuation string.

In embodiments, the blade may pivot relative to the first and second jaw members to cut tissue in response to actuation of the actuation string.

In certain embodiments, the actuation string may be coupled to a drive assembly that actuates actuation string.

In some embodiments, the end effector may be robotically controlled.

In embodiments, the actuation string may be positioned to move along a string guide supported in the second jaw member. The blade may be in the form of a blade chip supported on the string guide.

Other aspects, features, and advantages will be apparent from the description, the drawings, and the claims that follow.

### BRIEF DESCRIPTION OF DRAWINGS

The accompanying drawings, which are incorporated in and constitute a part of this specification, illustrate embodiments of the disclosure and, together with a general description of the disclosure given above and the detailed description given below, serve to explain the principles of the disclosure, wherein:
FIG. 1 is a perspective view of a robotic surgical system in accordance with the present disclosure;
FIG. 2 is an enlarged view of the indicated area of detail shown in FIG. 1 and illustrating one embodiment of an end effector of a surgical instrument of the robotic surgical system of FIG. 1 in unarticulated and articulated positions (with exemplary articulated positions illustrated in phantom);
FIG. 3 is a perspective view of the end effector of FIG. 2 with jaw members of the end effector shown in a clamped position and cabling thereof removed for clarity;
FIG. 4 is a perspective view, with parts separated, of the end effector of FIG. 3;
FIGS. 5 and 6 are enlarged views of the indicated areas of detail shown in FIG. 4;
FIG. 7 is a perspective view of a bottom jaw of the end effector of FIG. 3;
FIG. 8 is a perspective view of the end effector of FIG. 2 with the jaw members thereof shown in an unclamped position and a blade thereof shown in an unactuated position;
FIG. 9 is a perspective view of the end effector of FIG. 2 with portions thereof removed for clarity, and with the blade thereof shown in the unactuated position;
FIG. 10 is a cross-sectional view of the end effector of FIG. 2 as illustrated in FIG. 9 and taken along section line 10-10;
FIG. 11 is a perspective view of the end effector of FIG. 2 with portions thereof removed for clarity, and with the blade thereof shown in an actuated position;
FIG. 12 is a cross-sectional view of the end effector of FIG. 2 as illustrated in FIG. 11 and taken along section line 12-12;
FIG. 13 is a perspective view of another embodiment of an end effector of the robotic assembly of FIG. 1, the end effector shown with jaw members thereof in a clamped position;
FIG. 14 is a perspective view of the end effector of FIG. 13 with the jaw members thereof shown in an unclamped position;
FIG. 15 is a perspective view, with parts separated, of the end effector of FIGS. 13 and 14;
FIGS. 16 and 17 are enlarged views of the indicated areas of detail illustrated in FIG. 15;
FIG. 18 is a perspective view of the end effector of FIGS. 13 and 14 with portions thereof removed for clarity, and with a blade thereof shown in an unactuated position;
FIG. 19 is a cross-sectional view of the end effector of FIGS. 13 and 14 as illustrated in FIG. 18 and taken along section line 19-19;
FIG. 20 is a perspective view of the end effector of FIGS. 13 and 14 with portions thereof removed for clarity, and with a blade thereof shown in an actuated position;
FIG. 21 is a cross-sectional view of the end effector of FIGS. 13 and 14 as illustrated in FIG. 20 and taken along section line 21-21;
FIG. 22 is a perspective view of another embodiment of an end effector of the robotic assembly of FIG. 1, the end effector shown with jaw members thereof in a clamped position; and
FIGS. 23 - 26 are progressive views illustrating a blade of the end effector of FIG. 22 moving from an unactuated position to an actuated position.

### DETAILED DESCRIPTION

Embodiments of the present disclosure are described in detail with reference to the drawings, in which like reference numerals designate identical or corresponding elements in each of the several views. As commonly known, the term "clinician" refers to a doctor, a nurse, or any other care provider and may include support personnel. Additionally, the term "proximal" refers to the portion of structure that is closer to the clinician and the term "distal" refers to the portion of structure that is farther from the clinician. In addition, directional terms such as front, rear, upper, lower, top, bottom, and the like are used simply for convenience of description and are not intended to limit the disclosure attached hereto.

In the following description, well-known functions or constructions are not described in detail to avoid obscuring the present disclosure in unnecessary detail.

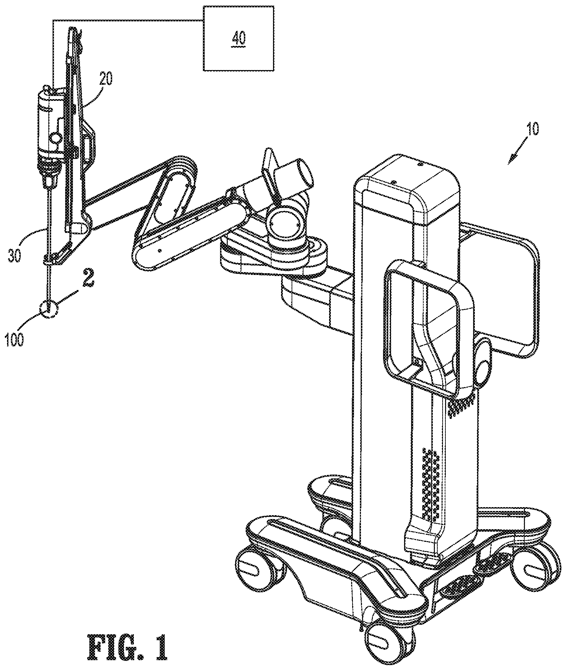

With brief reference to FIG. 1, a robotic surgical system 10 includes a robotic arm 20 that supports a wristed surgical instrument 30 having an end effector 100. Surgical instrument 30 is disposed in electrical communication with an electrosurgical energy source 40 such as a generator. For a detailed description of the construction and operation of an example generator, reference may be made to U.S. Patent Application Publication No. 20140171935, entitled "System and Method for Voltage and Current Sensing,". Robotic surgical system 10 employ various robotic elements to assist the clinician and allow remote operation (or partial remote operation) of surgical instrumentation such as surgical instrument 30. Various robotic arms, gears, cams, pulleys, electric and mechanical motors, etc. may be employed for this purpose and may be designed with robotic surgical system 10 to assist the clinician during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

Robotic surgical system 10 may be employed with one or more consoles (not shown) that are next to the operating theater or located in a remote location. In this instance, one team of clinicians may prep the patient for surgery and configure the robotic surgical system with one or more of the instruments/end effectors disclosed herein while another clinician (or group of clinicians) remotely controls the instruments/end effectors via robotic surgical system 10. As can be appreciated, a highly skilled clinician may perform multiple operations in multiple locations without leaving his/her remote console which can be both economically advantageous and a benefit to the patient or a series of patients. For a detailed description of exemplary medical work stations and/or components thereof, reference may be made to U.S. Patent Application Publication No. 2012/0116416 and PCT Application Publication No. WO2016/025132.

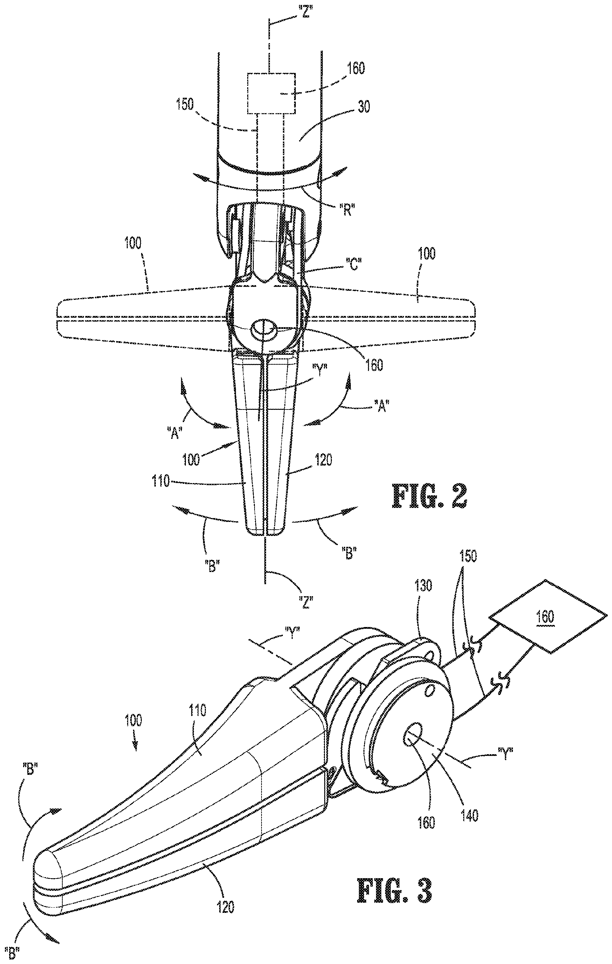

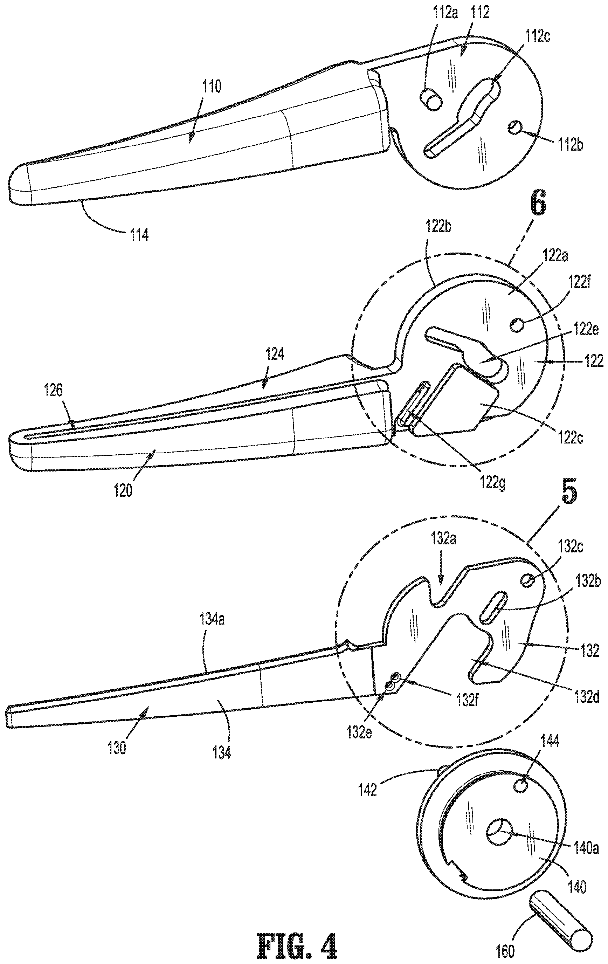

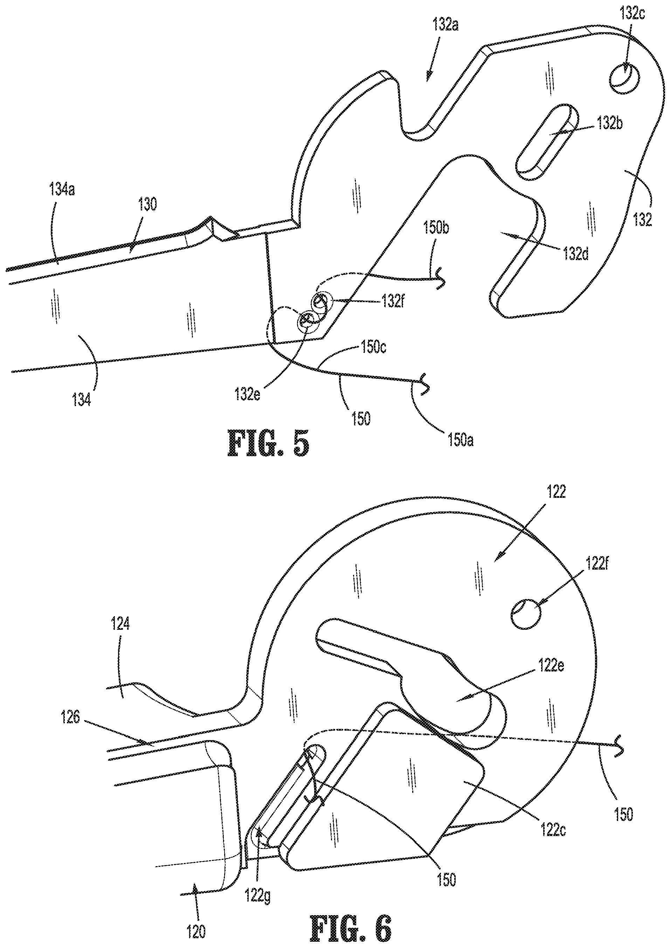

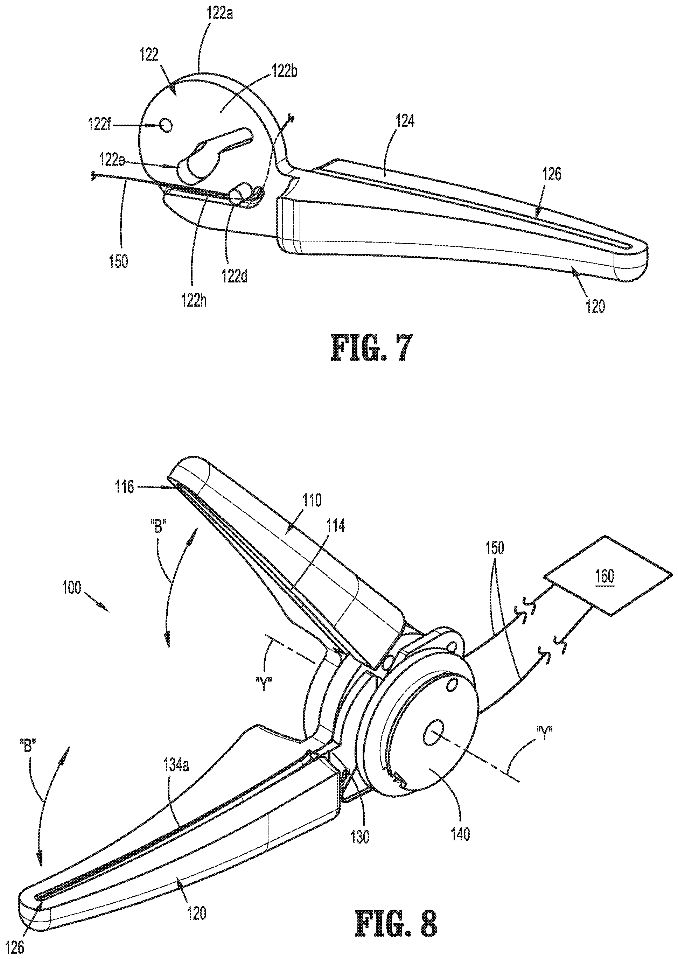

Turning now to FIGS. 2-12, end effector 100 of surgical instrument 30 extends distally from surgical instrument 30 and defines a longitudinal axis "Z-Z" about which end effector 100 can rotate, as indicated by arrows "R." End effector 100 includes a top or first jaw member 110, a bottom or second jaw member 120 coupled to first jaw member 110, a blade 130 supported between first and second jaw members 110, 120, a force multiplier 140, one or more actuation strings 150 coupled to blade 130, and a pivot pin 160. First and second jaw members 110, 120 are positioned to articulate and pivot about an axis "Y-Y" defined by pivot pin 160. Axis "Y-Y" extends transverse (e.g., perpendicular) to longitudinal axis "Z-Z." In particular, first and second jaw members 110, 120 of end effector 100 are positioned to articulate together about axis "Y-Y," as indicated by arrows "A" seen in FIG. 2, between an unarticulated position relative to longitudinal axis "Z-Z" (as illustrated in FIG. 2), and one or more articulated positions relative to longitudinal axis "Z-Z" (illustrated in FIG. 2 by the phantom lines). And first and second jaw members 110, 120 are positioned to pivot about axis "Y-Y," as indicated by arrows "B" in FIGS. 3 and 8, between a clamped position (FIG. 3) and one or more unclamped positions (FIG. 8). End effector 100 is coupled to one or more cables "C" of surgical instrument 30 that are robotically actuatable to impart such articulation and/or pivoting movement to end effector 100, for instance through force multiplier 140. For a more detailed description of such articulation and/or pivoting movement and structure, reference can be made to U.S. Patent Application Publication No. 2017/0231653, entitled "Robotically Controlling Mechanical Advantage Gripping," of which one or more components or features thereof can be modified for use with the presently disclosed end effectors.

First jaw member 110 of end effector 100 includes a first cam plate 112 on a proximal end portion thereof, and a first tissue contact surface 114 that extends distally from first cam plate 112. First tissue contact surface 114 defines a first knife channel 116 therein. First cam plate 112 of first jaw member 110 includes a pin 112a and defines a pin hole 112b and a pin slot 112c therethrough. First tissue contact surface 114 of first jaw member 110 is electrically coupled to an electrosurgical energy source 40 (FIG. 1) to enable first tissue contact surface 114 to selectively seal tissue in contact therewith. For a detailed description of the construction and operation of a vessel sealer with a similar tissue contact surface, reference may be made to U.S. Patent No. 7,101,371, entitled "Vessel Sealer and Divider,".

Second jaw member 120 of end effector 100 includes a second cam plate 122 on a proximal end portion thereof, and a second tissue contact surface 124 that extends distally from second cam plate 122. Second tissue contact surface 124 defines a second knife channel 126 therein. Like first tissue contact surface 114 of first jaw member 110, second tissue contact surface 124 of second jaw member 120 is disposed in electrical communication with electrosurgical energy source 40 to selectively seal tissue that contacts second tissue contact surface 124. Second cam plate 122 of second jaw member 120 includes a first side 122a and a second side 122b. Second cam plate 122 includes a ramp 122c that extends from first side 122a of second cam plate 122 and a pin 122d that extends from second side 122b of second cam plate 122. Although ramp 122c is shown with a trapezoidal configuration, ramp 122c may have any suitable configuration such as rectangular or wedge configuration. Second cam plate 122 defines a pin slot 122e, a pin hole 122f, and a string slot 122g therethrough. Second cam plate 122 further defines a string channel 122h in second side 122b of second cam plate 122 that is disposed in registration with string slot 122g so that string slot 122g and string channel 122h are positioned to receive actuation string(s) 150. Actuation string(s) 150 can be fixedly secured to second cam plate 122 within string slot 122g and string channel 122h by an suitable fastening technique such as adhesion, crimping, friction-fitting, etc., or combinations thereof.

Blade 130 of end effector 100 includes a third cam plate 132 on a proximal end portion thereof and a cutting arm 134 that extends distally from third cam plate 132. Third cam plate 132 of blade 130 defines a pin notch 132a, pin slot 132b, a pin hole 132c, a ramp slot 132d, and first and second string holes 132e, 132f therethrough. Cutting arm 134 of blade 130 is movable through first and second knife channels 116, 126 of first and second jaw members 110, 120, and includes a cutting edge 134 configured to cut tissue.

Force multiplier 140 of end effector 100 defines a pin opening 140a therethrough. Force multiplier 140 includes pins 142, 144 that extend therefrom. Pivot pin 160 is receivable through pin opening 140a of force multiplier 140 and pin slots 132b, 122e, and 112c of blade 130, second jaw member 120, and first jaw member 110, respectively, to movably couple force multiplier 140, blade 130, and first and second jaw members 110, 120 together.

Actuation string 150 of end effector 100 includes opposite ends 150a, 150b and a central portion 150c that extends between opposite end portions 150a, 150b of actuation string 150. Actuation string 150 is routed back and forth through string holes 132e, 132f of blade 132. First end portion 150a of actuation string 150 extends through string slot 122g of cam plate 122 of second jaw member 120 and proximally to a drive assembly 160 supported in surgical instrument 30 (FIG. 1). Second end portion 150b of actuation string 150 extends through string channel 122h of cam plate 122 of second jaw member 120 and proximally to drive assembly 160.

Drive assembly 160 of surgical instrument 30 is configured to actuate actuation string 150 of end effector 100 of surgical instrument 30. Drive assembly 160 can have any suitable mechanical and/or electrical component to effectuate actuation of actuation string (e.g., screws, nuts, gears, pulleys, circuitry, controllers, motors, etc., or combinations thereof).

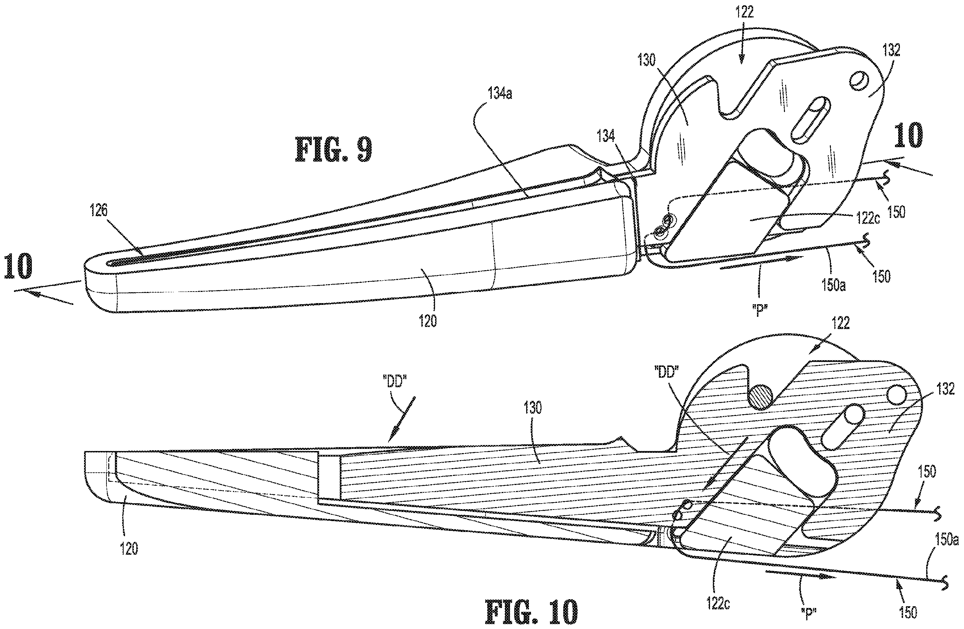

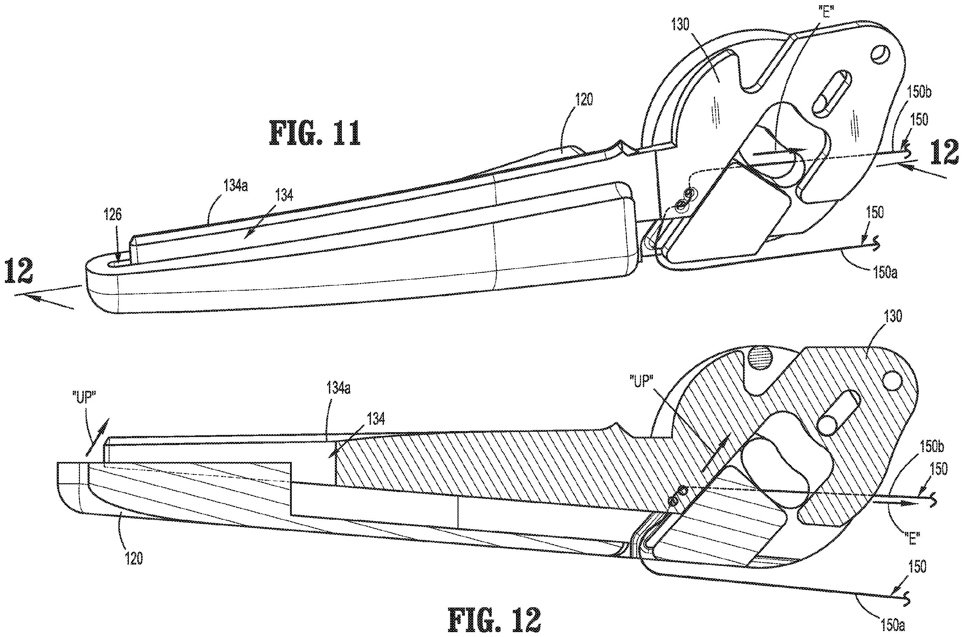

As seen in FIGS. 2-12, in operation, to actuate blade 130 of end effector 100 between the unactuated position (FIGS. 9 and 10) and the actuated position (FIGS. 11 and 12), opposite end portions of actuation string 150 are moved in opposite directions (e.g., axially; proximal-distal) such that blade 130 moves vertically and axially. For example, to urge blade 130 toward the unactuated position, (e.g., so third cam plate 132 of blade 130 moves downwardly and distally along ramp 122c of second jaw member 120, as indicated by arrows "DD") a first end portion 150a of actuation string 150 is drawn proximally, as indicated by arrows "P." And to urge blade 130 toward the actuated position (e.g., so third cam plate 132 of blade 130 moves upwardly and proximally along ramp 122c of second jaw member 120, as indicated by arrows "UP"), second end portion 150b of actuation string 150 is drawn proximally, as indicated by arrows "E." As blade 130 moves from the unactuated position to the actuated position, cutting edge 134a of cutting arm 134 severs tissue grasped between first and second jaw members 110, 120 as cutting arm 134 moves vertically through knife channels 116, 126 of first and second jaw members 110, 120.

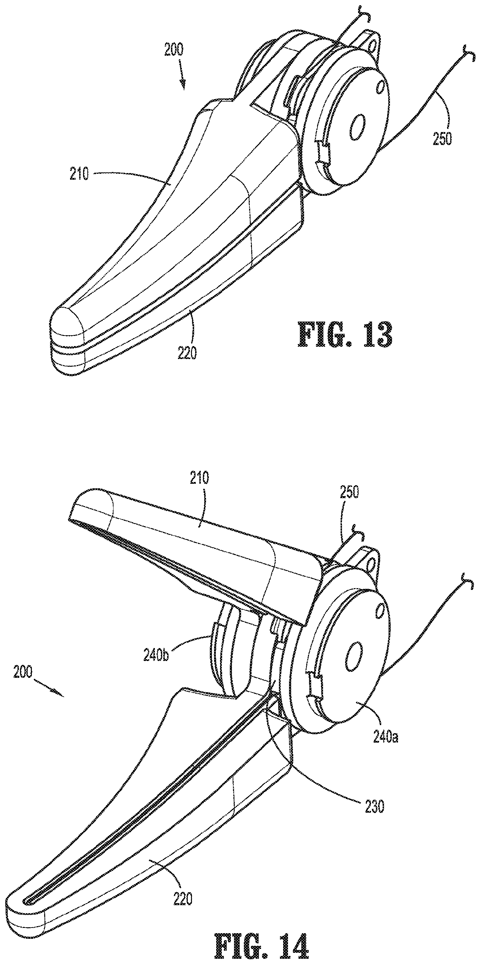

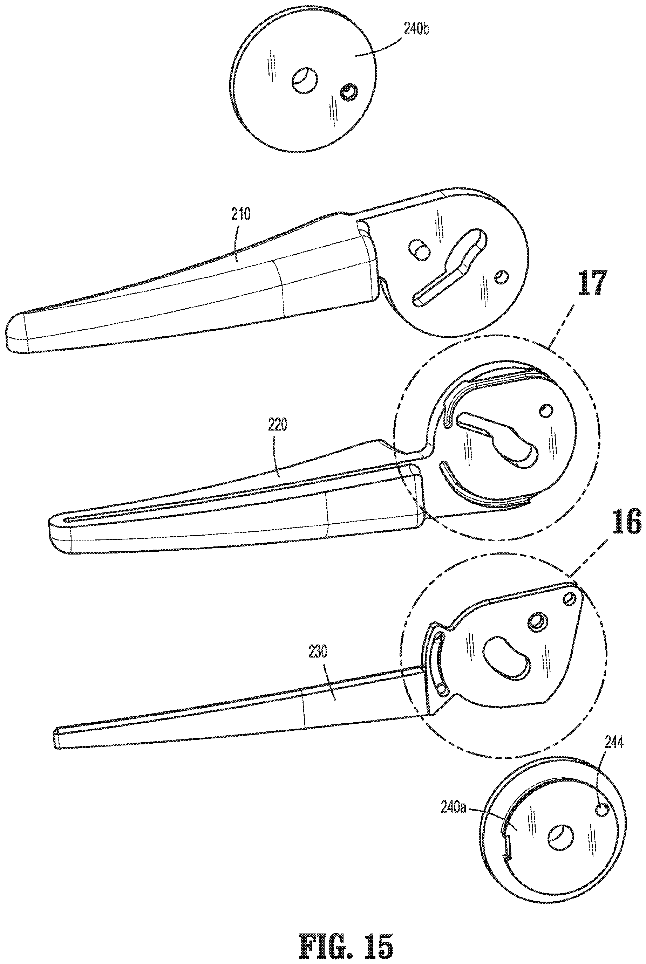

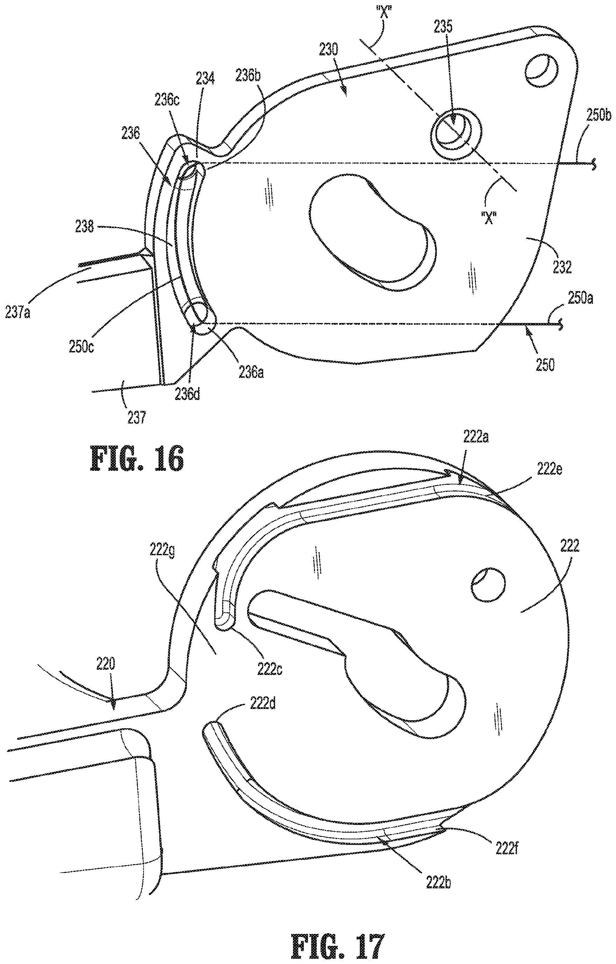

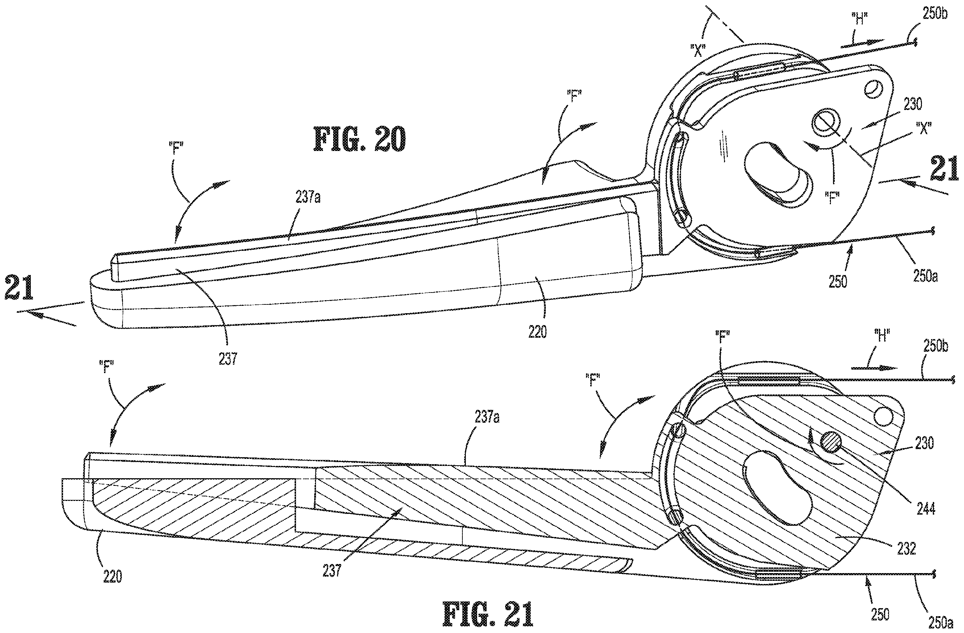

Turning now to FIGS. 13-21, another embodiment of an end effector 200 includes a first jaw member 210, a second jaw member 220, a blade 230, a first force multiplier 240a, a second force multiplier 240b, and an actuation string 250. First jaw member 210 of end effector 200 is similar to first jaw member 110 of end effector 100, and first and second force multipliers 240a, 240b of end effector 200 are similar to force multiplier 140 of end effector 100. Second jaw member 220 of end effector 200 is similar to second jaw member 120 of end effector 100, but includes a cam plate 222 on a proximal end portion thereof that defines first and second string channels 222a with curvilinear configurations. First and second string channels 222a, 222b have inner end portions 222c, 222d, respectively, that curve toward one another along a distal portion of cam plate 222, and outer end portions 222e, 222f, respectively, that extend parallel to one another and open at opposite end portions of a proximal end portion of cam plate 220. Inner end portions 222c, 222d of respective first and second string channels 222a, 222b are separated by a gap portion 222g. Blade 230 of end effector 200 is also similar to blade 130 of end effector 100 but includes a cam plate 232 having a bridge portion 234 supported on a distal portion thereof. Cam plate 232 defines a pivot hole 235 therethrough. Pivot hole 235 defines a blade pivot axis "X-X" therethrough that receives a pin 244 from force multiplier 240a therein. Bridge portion 234 defines a string cutout 236 that has an arched profile from a lower portion 236a thereof to an upper portion 236b thereof. String cutout 236 further defines an upper opening 236c formed in upper portion 236b thereof, and a lower opening 236d formed in lower portion 236a thereof. Bridge portion 234 further includes a bridge 238 that extend between upper and lower openings 236c, 236d and follows the arched profile of string cutout 236. Blade 230 further includes a cutting arm 237 that extends distally from cam plate 232 and includes a cutting edge 237a.

Actuation string 250 of end effector 200 is similar to actuation string 150 of end effector 100, but includes first end portion 250a, a second end portion 250b and a central portion 250c that extends between first and second end portions 205a, 250b. Central portion 250c is secured to bridge 238 of cam plate 232 of blade 230 with first end portion 250a of actuation string 250 routed through lower opening 236d of string cutout 236, and second end portion 250a of actuation string 250 routed through upper opening 236c of string cutout 236. Central portion 250c can be secured to bridge 238 of cam plate 232 using any suitable fastening techniques such as adhesion, crimping, friction-fitting, etc., or combinations thereof. From string cutout 236, first and second end portions 250a, 250b extend proximally and couple to drive assembly 160 to enable drive assembly 160 to selectively actuate (e.g., extend and/or release) first and/or second end portions 205a, 250b of actuation string 250. End effector 200 further includes first and second retaining tubes 270a, 270b supported in first and second string channels 222a, 222b, respectively, of cam plate 222 of second jaw member 220. First and second retaining tubes 270a, 270b guide first and second end portions 250a, 250b, respectively, of actuation string 250 therethrough.

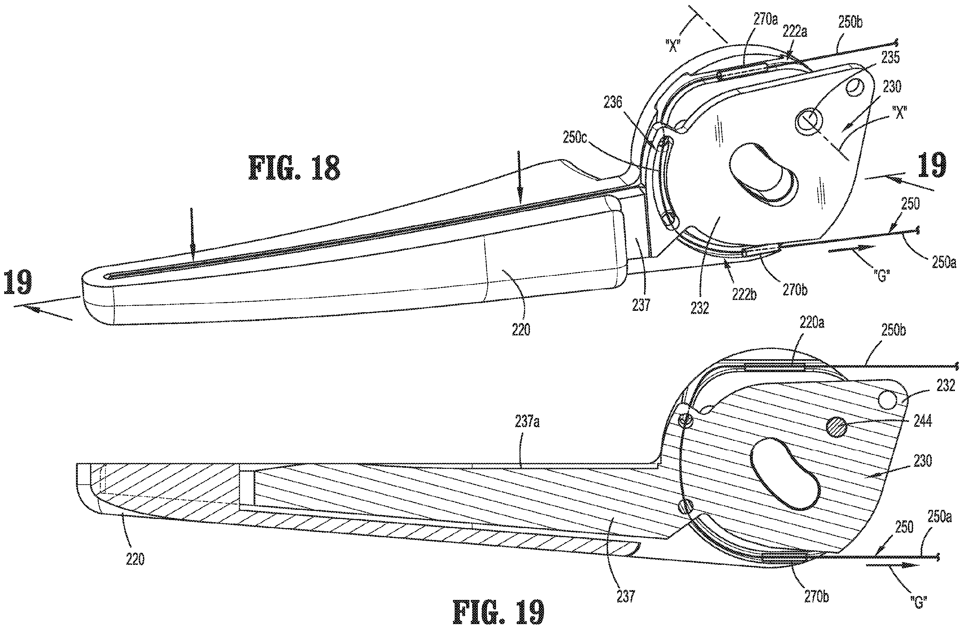

In operation, as best seen FIGS. 18-21, actuation of actuation string 250, as indicated by arrows "G" or "H" causes blade 230 to rotate about pivot axis "X-X," as indicated by arrows "F," so that cutting arm 237 of blade 230 can sever tissue grasped between first and second jaw members 210, 220 when end effector 300 is disposed in the clamped position (FIG. 13). In this embodiment, a distal portion of cutting arm 237 moves farther, relative to first and/or second jaw members 210, 220, than a proximal portion thereof (e.g., to due to the difference in radius length from the pivot point) as blade rotates about pivot axis "X-X."

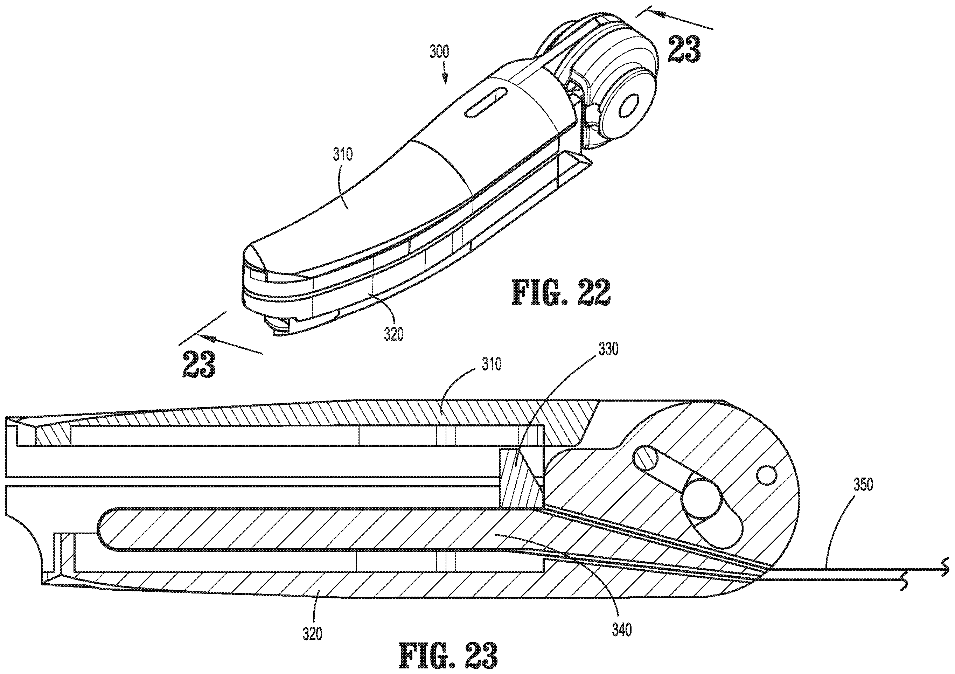

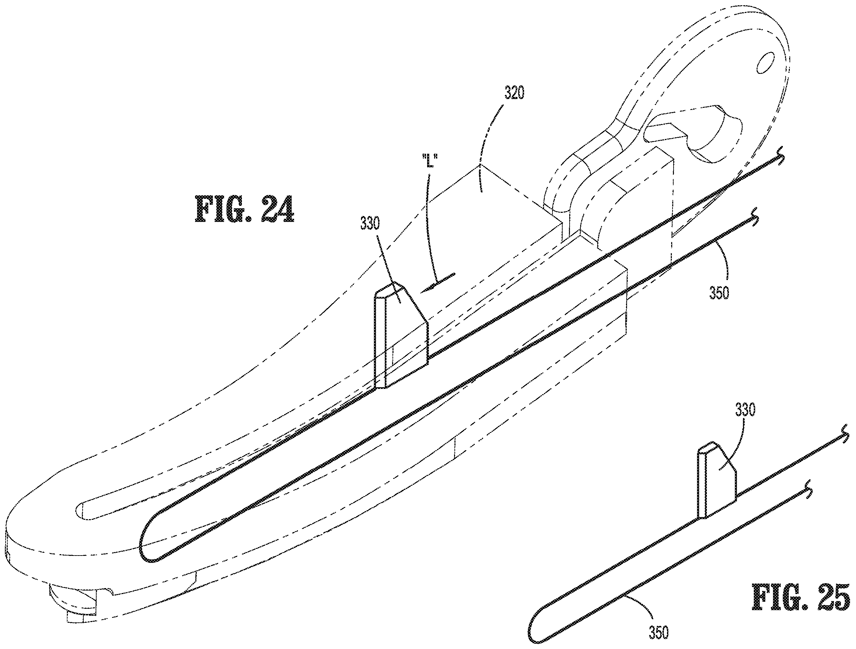

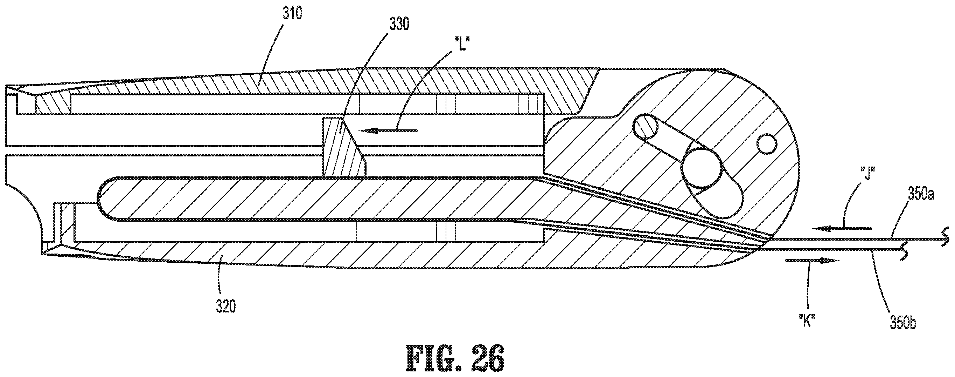

With reference now to FIGS. 22-26, another embodiment of an end effector 300 includes a first jaw member 310 and a second jaw member 320 that are pivotally coupled together between clamped and unclamped positions. End effector 300 includes a blade 330 that is secured to an actuation string 350. Actuation string 350 is slidably supported on a string guide 340 supported in second jaw member 320. Blade 330 is in the form of a blade chip supported on string guide 340.

In operation, actuation string 350 is advanced along string guide 340 as opposite end portions 350a, 350b of actuation string 350 are moved relative to one another, as indicated by arrows "J" and "K," to axially advance blade 330 through first and second jaw members 310, 320, as indicated by arrow "L."

Advantageously, the actuation string of the presently disclosed end effectors enables blade deployment through a robotic wristed instrument. End effectors 100 and 200, in particular, provide short throw blade motion that simplify complexity of drive motion components.

The actuation string of the present disclosure may have any suitable configuration such as fibers, cables, ropes, chains, etc., or combinations thereof. For instance, the actuation string may include a high tensile fiber. In certain embodiments, string, or portions thereof, may include one or more coatings such as a lubricious coating for reducing friction.

In some embodiments, the retaining function of retaining tubes 270a, 270b of end effector 200 can be achieved with, for example, an overmolded channel and/or a catch formed in cam plate 222 using any suitable technique (e.g., stamping or the like), each or both of which may be used in conjunction with or in place of retaining tubes 270a, 270b.

In certain embodiments, to control motion of blade 130, in addition to, or in place of ramp 122c of cam plate 122 of jaw member 120, one or more pins/extrusions may extend from cam plate 122 of jaw member 120 that can cooperate with holes/cutouts defined in blade 130 (e.g., cam plate 132 thereof). Alternatively, or additionally, such pins/extrusions may extend from blade 130 and cooperate with holes/cutouts defined in jaw member 120 (e.g., cam plate 122 thereof).

In embodiments, one or more of the force multipliers may cooperate with jaw members and/or blades (e.g., via cutouts, holes, pins, etc., or combinations thereof) to prevent (e.g., hard stop) the presently disclosed blades from deploying when the jaw members are in the unclamped position.

As can be appreciated, securement of any of the components of the presently disclosed apparatus can be effectuated using known securement techniques such welding, crimping, gluing, fastening, etc.

Persons skilled in the art will understand that the structures and methods specifically described herein and illustrated in the accompanying figures are non-limiting exemplary embodiments, and that the description, disclosure, and figures should be construed merely as exemplary of particular embodiments. It is to be understood, therefore, that the present disclosure is not limited to the precise embodiments described, and that various other changes and modifications may be effected by one skilled in the art without departing from the scope of the claims.

## Claims

1. An end effector (100) for a wristed surgical instrument (30), the end effector (100) comprising:
a first jaw member (110);
a second jaw member (120) coupled to the first jaw member (110) by a pivot pin (160), the first and second jaw members (110, 120) positioned to pivot and articulate about a pivot axis defined by the pivot pin (160);
an actuation string (150); and
a blade (130) including a cam plate (132) supported between the first and second jaw members (110, 120) and secured to the actuation string (150), the blade (130) movable relative to the first and second jaw members (110, 120) to sever tissue clamped between the first and second jaw members (110, 120) in response to actuation of the actuation string (150), the actuation string (150) being secured to the cam plate (132);
**characterised in that**
the second jaw member (120) includes a cam plate (122) and the pivot pin (160) is positioned through the cam plates (122, 132) of the blade (130) and the second jaw member (120).

2. The end effector (100) of claim 1, wherein the actuation string (150) is routed through the cam plate (132) of the blade (130).

3. The end effector (100) of claim 1, wherein the cam plate (122) of the second jaw member (120) includes a ramp (122c), the cam plate (132) of the blade (130) positioned to move along the ramp (122c).

4. The end effector (100) of claim 3, wherein the cam plate (132) of the blade (130) defines a ramp slot (132d) that receives the ramp (122c) of the second jaw member (120); preferably wherein the blade (130) is positioned to move vertically and axially relative to the second jaw member (120) as the cam plate (132) of the blade (130) cams along the ramp (122c) of the second jaw member (120).

5. The end effector (100) of claim 1, wherein the blade (130) includes a cutting arm (134) that extends distally from the cam plate (132) of the blade (130); preferably wherein the blade (130) is configured to pivot relative to the second jaw member (120) in response to actuation of the actuation string (150).

6. The end effector (100) of claim 5, wherein cutting arm (134) includes a distal portion and a proximal portion, the distal portion of the cutting arm (134) configured to move farther than the proximal portion of the cutting arm (134) as the blade (130) pivots relative to the second jaw member (120).

7. A wristed surgical instrument (30) comprising:
an end effector (100) according to any of claims 1 to 6, defining a longitudinal axis and a pivot axis transverse to the longitudinal axis, the first jaw member (110) and the second jaw member (120) being movable about the pivot axis between an unclamped position and a clamped position and the actuation string (150) being axially movable to actuate the blade (130) relative to the first and second jaw members (110, 120) when the first and second jaw members (110, 120) are in the clamped position.

8. The wristed surgical instrument (30) of claim 7, wherein the end effector (100) is coupled to an electrosurgical energy source (40).

9. The wristed surgical instrument (30) of claim 7 or claim 8, wherein the blade (130) is configured to move from the second jaw member (120) toward the first jaw member (110) to cut tissue.

10. The wristed surgical instrument (30) of claim 9, wherein the blade (130) is vertically and axially movable relative to the first and second jaw members (110, 120).

11. The wristed surgical instrument (30) of claim 10, wherein the blade (130) cams along the ramp (122c) of the second jaw member (120) in response to axial translation of the actuation string (150).

12. The wristed surgical instrument (30) of any of claims 7 to 11, wherein the actuation string (150) is coupled to a drive assembly that actuates actuation string (150).

13. The wristed surgical instrument (30) of any of claims 7 to 12, wherein the end effector (100) is robotically controlled.

14. The wristed surgical instrument (30) of any of claims 7 to 13, wherein the actuation string (150) is positioned to move along a string guide (340) supported in the second jaw member (120).

15. The wristed surgical instrument (30) of any of claims 7 to 14, wherein the blade (130) is in the form of a blade chip supported on the string guide (340).

## Patentansprüche

1. Endeffektor (100) für ein chirurgisches Handgelenksinstrument (30), der Endeffektor (100) umfassend:
ein erstes Backenelement (110);
ein zweites Backenelement (120), das mit dem ersten Backenelement (110) durch einen Schwenkstift (160) gekoppelt ist, wobei das erste und das zweite Backenelement (110, 120) positioniert sind, um um eine Schwenkachse herum zu schwenken und gelenkig zu verbinden, die durch den Schwenkstift (160) definiert ist;
eine Betätigungsschnur (150); und
eine Klinge (130), die eine Nockenplatte (132) einschließt, die zwischen dem ersten und dem zweiten Backenelement (110, 120) getragen und an der Betätigungsschnur (150) befestigt ist, wobei die Klinge (130) relativ zu dem ersten und dem zweiten Backenelement (110, 120) bewegbar ist, um Gewebe, das zwischen dem ersten und dem zweiten Backenelement (110, 120) eingeklemmt ist, als Reaktion auf eine Betätigung der Betätigungsschnur (150) zu durchtrennen, wobei die Betätigungsschnur (150) an der Nockenplatte (132) befestigt ist;
**dadurch gekennzeichnet, dass**
das zweite Backenelement (120) eine Nockenplatte (122) einschließt und der Schwenkstift (160) durch die Nockenplatten (122, 132) der Klinge (130) und das zweite Backenelement (120) hindurch positioniert ist.

2. Endeffektor (100) nach Anspruch 1, wobei die Betätigungsschnur (150) durch die Nockenplatte (132) der Klinge (130) geführt ist.

3. Endeffektor (100) nach Anspruch 1, wobei die Nockenplatte (122) des zweiten Backenelements (120) eine Rampe (122c) einschließt, wobei die Nockenplatte (132) der Klinge (130) positioniert ist, um sich entlang der Rampe (122c) zu bewegen.

4. Endeffektor (100) nach Anspruch 3, wobei die Nockenplatte (132) der Klinge (130) einen Rampenschlitz (132d) definiert, der die Rampe (122c) des zweiten Backenelements (120) aufnimmt; vorzugsweise wobei die Klinge (130) positioniert ist, um sich vertikal und axial relativ zu dem zweiten Backenelement (120) zu bewegen, wenn die Nockenplatte (132) der Klinge (130) entlang der Rampe (122c) des zweiten Backenelements (120) mit Nocken versehen ist.

5. Endeffektor (100) nach Anspruch 1, wobei die Klinge (130) einen Schneidarm (134) einschließt, der sich von der Nockenplatte (132) der Klinge (130) distal erstreckt; vorzugsweise wobei die Klinge (130) konfiguriert ist, um als Reaktion auf die Betätigung der Betätigungsschnur (150) relativ zu dem zweiten Backenelement (120) zu schwenken.

6. Endeffektor (100) nach Anspruch 5, wobei der Schneidarm (134) einen distalen Abschnitt und einen proximalen Abschnitt einschließt, wobei der distale Abschnitt des Schneidarms (134) konfiguriert ist, um sich weiter als der proximale Abschnitt des Schneidarms (134) zu bewegen, wenn die Klinge (130) relativ zu dem zweiten Backenelement (120) schwenkt.

7. Chirurgisches Handgelenksinstrument (30), umfassend:
einen Endeffektor (100) nach einem der Ansprüche 1 bis 6, der eine Längsachse und eine Schwenkachse quer zu der Längsachse definiert, wobei das erste Backenelement (110) und das zweite Backenelement (120) um die Schwenkachse herum zwischen einer uneingeklemmten Position und einer eingeklemmten Position bewegbar sind und die Betätigungsschnur (150) axial bewegbar ist, um die Klinge (130) relativ zu dem ersten und dem zweiten Backenelement (110, 120) zu betätigen, wenn sich das erste und das zweite Backenelement (110, 120) in der eingeklemmten Position befinden.

8. Chirurgisches Handgelenksinstrument (30) nach Anspruch 7, wobei der Endeffektor (100) mit einer elektrochirurgischen Energiequelle (40) gekoppelt ist.

9. Chirurgisches Handgelenksinstrument (30) nach Anspruch 7 oder 8, wobei die Klinge (130) konfiguriert ist, um sich von dem zweiten Backenelement (120) zu dem ersten Backenelement (110) hin zu bewegen, um Gewebe zu schneiden.

10. Chirurgisches Handgelenksinstrument (30) nach Anspruch 9, wobei die Klinge (130) relativ zu dem ersten und dem zweiten Backenelement (110, 120) vertikal und axial bewegbar ist.

11. Chirurgisches Handgelenksinstrument (30) nach Anspruch 10, wobei die Klinge (130) entlang der Rampe (122c) des zweiten Backenelements (120) als Reaktion auf eine axiale Verschiebung der Betätigungsschnur (150) mit Nocken versehen ist.

12. Chirurgisches Handgelenksinstrument (30) nach einem der Ansprüche 7 bis 11, wobei die Betätigungsschnur (150) mit einer Antriebsbaugruppe gekoppelt ist, die die Betätigungsschnur (150) betätigt.

13. Chirurgisches Handgelenksinstrument (30) nach einem der Ansprüche 7 bis 12, wobei der Endeffektor (100) robotisch gesteuert wird.

14. Chirurgisches Handgelenksinstrument (30) nach einem der Ansprüche 7 bis 13, wobei die Betätigungsschnur (150) positioniert ist, um sich entlang einer Schnurführung (340) zu bewegen, die in dem zweiten Backenelement (120) getragen wird.

15. Chirurgisches Handgelenksinstrument (30) nach einem der Ansprüche 7 bis 14, wobei die Klinge (130) in Form eines Klingenspans ist, der an der Schnurführung (340) getragen wird.

## Revendications

1. Effecteur terminal (100) pour un instrument chirurgical à poignée (30), l'effecteur terminal (100) comprenant :
un premier élément de mâchoire (110) ;
un second élément de mâchoire (120) accouplé au premier élément de mâchoire (110) par une goupille de pivot (160), le premier et le second élément de mâchoire (110, 120) étant positionnés pour pivoter et s'articuler autour d'un axe de pivotement défini par la goupille de pivot (160) ;
une tige d'actionnement (150) ; et
une lame (130) comportant une plaque de came (132) supportée entre le premier et le second élément de mâchoire (110, 120) et fixée à la tige d'actionnement (150), la lame (130) pouvant être déplacée par rapport au premier et au second élément de mâchoire (110, 120) pour couper le tissu enserré entre le premier et le second élément de mâchoire (110, 120) en réponse à l'actionnement de la tige d'actionnement (150), la tige d'actionnement (150) étant fixée à la plaque de came (132) ;
**caractérisé en ce que**
le second élément de mâchoire (120) comporte une plaque de came (122) et la goupille de pivot (160) est positionnée à travers les plaques de came (122, 132) de la lame (130) et le second élément de mâchoire (120).

2. Effecteur terminal (100) selon la revendication 1, dans lequel la tige d'actionnement (150) est acheminée à travers la plaque de came (132) de la lame (130).

3. Effecteur terminal (100) selon la revendication 1, dans lequel la plaque de came (122) du second élément de mâchoire (120) comporte une rampe (122c), la plaque de came (132) de la lame (130) étant positionnée pour se déplacer le long de la rampe (122c).

4. Effecteur terminal (100) selon la revendication 3, dans lequel la plaque de came (132) de la lame (130) définit une fente de rampe (132d) qui reçoit la rampe (122c) du second élément de mâchoire (120) ; de préférence dans lequel la lame (130) est positionnée pour se déplacer verticalement et axialement par rapport au second élément de mâchoire (120) lorsque la plaque de came (132) de la lame (130) se déplace le long de la rampe (122c) du second élément de mâchoire (120).

5. Effecteur terminal (100) selon la revendication 1, dans lequel la lame (130) comporte un bras de coupe (134) qui s'étend de manière distale à partir de la plaque de came (132) de la lame (130) ; de préférence dans lequel la lame (130) est conçue pour pivoter par rapport au second élément de mâchoire (120) en réponse à l'actionnement de la tige d'actionnement (150).

6. Effecteur terminal (100) selon la revendication 5, dans lequel le bras de coupe (134) comporte une partie distale et une partie proximale, la partie distale du bras de coupe (134) étant conçue pour se déplacer plus loin que la partie proximale du bras de coupe (134) lorsque la lame (130) pivote par rapport au second élément de mâchoire (120).

7. Instrument chirurgical à poignée (30) comprenant :
un effecteur terminal (100) selon l'une quelconque des revendications 1 à 6, définissant un axe longitudinal et un axe de pivotement transversal à l'axe longitudinal, le premier élément de mâchoire (110) et le second élément de mâchoire (120) étant mobiles autour de l'axe de pivotement entre une position non serrée et une position serrée et la tige d'actionnement (150) étant mobile axialement pour actionner la lame (130) par rapport au premier et au second élément de mâchoire (110, 120) lorsque le premier et le second élément de mâchoire (110, 120) sont dans la position serrée.

8. Instrument chirurgical à poignée (30) selon la revendication 7, dans lequel l'effecteur terminal (100) est accouplé à une source d'énergie électrochirurgicale (40).

9. Instrument chirurgical à poignée (30) selon la revendication 7 ou la revendication 8, dans lequel la lame (130) est conçue pour se déplacer du second élément de mâchoire (120) vers le premier élément de mâchoire (110) pour couper le tissu.

10. Instrument chirurgical à poignée (30) selon la revendication 9, dans lequel la lame (130) est mobile verticalement et axialement par rapport au premier et au second élément de mâchoire (110, 120).

11. Instrument chirurgical à poignée (30) selon la revendication 10, dans lequel la lame (130) se déplace le long de la rampe (122c) du second élément de mâchoire (120) en réponse à une translation axiale de la tige d'actionnement (150).

12. Instrument chirurgical à poignée (30) selon l'une quelconque des revendications 7 à 11, dans lequel la tige d'actionnement (150) est accouplée à un ensemble d'entraînement qui actionne la tige d'actionnement (150).

13. Instrument chirurgical à poignée (30) selon l'une quelconque des revendications 7 à 12, dans lequel l'effecteur terminal (100) est commandé par robot.

14. Instrument chirurgical à poignée (30) selon l'une quelconque des revendications 7 à 13, dans lequel la tige d'actionnement (150) est positionnée pour se déplacer le long d'un guide de tige (340) supporté dans le second élément de mâchoire (120).

15. Instrument chirurgical à poignée (30) selon l'une quelconque des revendications 7 à 14, dans lequel la lame (130) est sous la forme d'une puce de lame supportée sur le guide de tige (340).
